# EUROPÄISCHE PATENTSCHRIFT

(11) **EP 2 131 789 B1**
(45) Veröffentlichungstag und Bekanntmachung des Hinweises auf die Patenterteilung: **11.06.2014**
(21) Anmeldenummer: 08709203.7
(22) Anmeldetag: 25.02.2008
(51) Int. Cl.: A61F 2/34

(54) **REDUKTION DER ENTSTEHUNG VON STRIPE-WEAR AN INSERTS FÜR HÜFTGELENKPROTHESEN DURCH MODIFIZIERUNG DER GEOMETRIE DES ÜBERGANGS ZWISCHEN STIRNFLÄCHE UND KALOTTE**
REDUCTION OF THE DEVELOPMENT OF STRIPE WEAR ON INSERTS FOR HIP JOINT PROSTHESES BY MODIFYING THE GEOMETRY OF THE TRANSITION BETWEEN THE FACE AND SPHERICAL RECESS
RÉDUCTION DE L'APPARITION D'USURES DE TYPE "STRIPE-WEAR" SUR DES INSERTS DE PROTHÈSES DE LA HANCHE PAR MODIFICATION DE LA GÉOMÉTRIE DE LA TRANSITION ENTRE LA FACE FRONTALE ET LA CALOTTE

(30) Priorität: 23.02.2007 DE 102007009439
(43) Veröffentlichungstag der Anmeldung: 16.12.2009
(73) Patentinhaber: CeramTec GmbH, 73207 Plochingen (DE)
(72) Erfinder: PREUSS, Roman, 70771 Leinf.-Echterdingen (DE); PANDORF, Thomas, 73249 Wernau (DE)
(74) Vertreter: Uppena, Franz
(86) Internationale Anmeldenummer: PCT/EP2008/052238
(87) Internationale Veröffentlichungsnummer: WO 2008/102014

(56) Entgegenhaltungen:
- WO-A-95/23566
- WO-A-02/087476
- DE-U1- 29 601 522
- FR-A- 2 551 655
- US-A- 5 658 294
- US-A- 5 725 589

## Beschreibung

Für Hüftgelenkprothesen werden verschiedene Werkstoffe zur Realisierung einer biokompatiblen, verschleißarmen Lagerung verwendet. Dabei sind die so genannten Hart-Hart-Werkstoff-Paarungen gemäß Stand der Technik für eine dauerhafte und zuverlässige Versorgung des Patienten am besten geeignet. Bei diesen Werkstoff-Paarungen bestehen sowohl der Kugelkopf auf dem Hüftschaft als auch der Pfanneneinsatz in der Hüftpfanne aus einem im technischen Sinn harten Werkstoff. Es finden derzeit die Werkstoff-Paarungen Keramik-Keramik und Metall-Metall Anwendung. Aktuelle Untersuchungen lassen in Zukunft auch auf eine Verwendung der Werkstoff-Paarung Keramik-Metall schließen.

Bei andauernden hohen Belastungen von Hüftgelenkprothesen treten auch bei Verwendung der erwähnten harten Werkstoffe Verschleißerscheinungen auf. Diese führen zwar nicht zum Versagen, beispielsweise bei Prothesen aus keramischen Werkstoffen zum Bruch einer Komponente, sind aber dennoch unerwünscht. Der bei der Werkstoff-Paarung Metall-Metall durch die Reibung entstehende Abrieb kann für den menschlichen Körper schädlich sein.

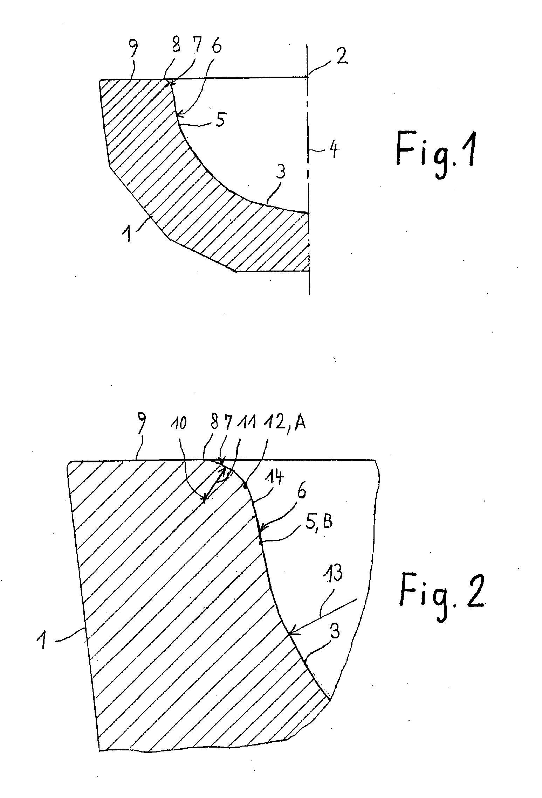

Die Figur 1 zeigt einen Schnitt durch einen Pfanneneinsatz 1 aus einem keramischen Werkstoff. Der Mittelpunkt 2 der Kalotte 3 liegt auf der Symmetrieachse 4 der Kalotte. Eine besondere Form des Verschleißes bei Hart-Hart-Werkstoff-Paarungen tritt im Bereich der so genannten Einlaufkante 5 auf. Diese befindet sich am Übergang zwischen der Kalotte 3 des Pfanneneinsatzes 1 und der Einlaufzone 6. Die Einlaufzone 6 endet mit einem Kreisbogen 7 tangential im Übergang 8 auf der Stirnfläche 9 des Pfanneneinsatzes 1. Durch Subluxation sowie auftretende Mikroseparation des Kugelkopfes kommt es zu hohen Belastungen im Bereich der Einlaufkante 5, sowohl für den Pfanneneinsatz 1 als auch für den hier nicht dargestellten Kugelkopf. Infolgedessen tritt lokal erhöhter Verschleiß auf, der, je nach Werkstoff, zu erhöhtem Abrieb oder zu sichtbarer Erhöhung der Oberflächenrauheit führt. Die als Stripe-Wear bezeichnete Erhöhung der Oberflächenrauheit ist ebenso unerwünscht, wie der ansonsten erhöht auftretende metallische Abrieb.

Der im Bereich der Einlaufkante 5 auftretende, erhöhte Verschleiß ist auf das Wirken hoher Flächenpressungen (Punktlast) sowie dem unstetigen Kraftverlauf bei der Gleitbewegung des Kugelkopfes im Bereich der Einlaufzone 6 zurückzuführen.

Der Erfindung liegt die Aufgabe zu Grunde, den Verschleiß im Bereich der Einlaufzone zu vermeiden oder zumindest stark zu reduzieren.

Zur Lösung dieser Aufgabe wird erfindungsgemäß vorgeschlagen, die Geometrie der Einlaufzone 6, zwischen dem Endpunkt 12 des Kreisbogens 7 und der Einlaufkante 5 in der Kalotte 3 als Kurve 14 auszuführen, deren Funktion an jedem Punkt zweifach stetig differenzierbar ist.

In Figur 2 ist als Ausschnitt im vergrößerten Maßstab gegenüber Figur 1 die Einlaufzone 6 im Pfanneinsatz 1 dargestellt. Der Kreisbogen 7 mit dem Mittelpunkt 10 am Beginn der Einlaufzone 6 hat einen Radius 11 mit der Länge R_{E}. Der Kreisbogen 7 endet im Punkt 12, wo sich die erfindungsgemäße Kurve 14 der Einlaufzone 6 anschließt. Der Übergang der Kurve 14 zum Kreisbogen der Kalotte 3 liegt in der Einlaufkante 5, wobei der Kreisbogen der Kalotte 3 einen Radius 13 mit der Länge R_{K} hat. Der Punkt A, der Beginn 12 des Kreisbogens 7, und der Punkt B, die Einlaufkante 5, sind die Punkte der Kurve 14, in denen ihre Krümmung mit der Krümmung der sich jeweils anschließenden Kurve, dem Kreisbogen 7 beziehungsweise dem Kreisbogen 3 der Kalotte, übereinstimmt.

Diese Kurve 14 der Einlaufzone 6 weist an ihren beiden Endpunkten A und B jeweils die Krümmung der sich anschließenden Kurve auf. Am Endpunkt A lautet die Formel der Kurve 14 also f" _{A}(x,y) = 1/R_{E} und am Endpunkt B f" B (x,y) = 1/R_{K}. Auf diese Weise ist der Krümmungsverlauf der Kurve 14, auf die sich der Kugelkopf zwischen Kalotte 3 und Einlaufradius 7 bewegt, stetig. Das führt dazu, dass der Verlauf sowohl der Kontaktkraft als auch der Flächenpressung ebenfalls stetig ist. Der Kugelkopf rollt in dem Pfanneneinsatz ab, Gleitbewegungen werden vermieden.

Zur mathematischen Beschreibung der Kurve 14 sind verschiedene Funktionen denkbar. Beispielhaft können an dieser Stelle die von Kurvengetrieben bekannten sinusoidalen Kurvenverläufe oder auch die Polynomformen genannt werden.

## Patentansprüche

1. Pfanneneinsatz einer Hüftgelenkprothese, in der der Kugelkopf des Oberschenkelknochens in der kalottenförmigen Ausnehmung des Pfanneneinsatzes gelagert ist und wobei diese Kalotte eine Einlaufzone aufweist, die auf der einen Seite mit einem Kreisbogen tangential in der Stirnfläche des Pfanneneinsatzes endet und auf der anderen Seite bis zur Einlaufkante reicht, **dadurch gekennzeichnet, dass** zwischen dem Beginn (12) des Kreisbogens (7) mit dem Radius (11) der Länge (R_{E}) und der Einlaufkante (5) der Kalotte (3) mit dem Radius (13) der Länge (R_{K}) die Einlaufzone (6) eine Kurve (14) bildet, deren Funktion an jedem Punkt zweifach stetig differenzierbar ist.

2. Pfanneneinsatz nach Anspruch 1, **dadurch gekennzeichnet, dass** die Kurve (14) in ihren jeweiligen Endpunkten (A), dem Endpunkt (12) des Kreisbogens (7), und (B), der Einlaufkante (5), die selbe Krümmung hat wie die sich an ihr anschließenden Kurven, der Kreisbogen (7) beziehungsweise die Kalotte (3).

3. Pfanneneinsatz nach Anspruch 1 oder 2, **dadurch gekennzeichnet, dass** der Verlauf der Kurve (14) sinusoidal ist oder eine Polynomform aufweist.

4. Pfanneneinsatz nach einem der Ansprüche 1 bis 3, **dadurch gekennzeichnet, dass** die Formel der Kurve (14) an ihrem Endpunkt (A), dem Beginn (12) des Kreisbogens (7), f"_{A}(x,y) = 1/R_{E} und an ihrem Endpunkt (B), der Einlaufkante (5) und dem Beginn des Kreisbogens der Kalotte (3), f" _{B} (x,y) = 1/R_{K} lautet.

## Claims

1. A socket insert of a hip joint prosthesis, in which the spherical head of the femur is mounted in the spherical-indentation-shaped recess of the socket insert and wherein this spherical indentation has a run-in zone which ends on the one side with a circular arc tangentially in the end face of the socket insert and extends on the other side as far as the run-in edge, **characterised in that** between the start (12) of the circular arc (7) with a radius (11) of length (R_{E}) and the run-in edge (5) of the spherical indentation (3) with a radius (13) of length (R_{K}) the run-in zone (6) forms a curve (14), the function of which can be continuously differentiated twice at every point.

2. A socket insert according to claim 1, **characterised in that** the curve (14) at its respective end points (A), the end point (12) of the circular arc (7), and (B), the run-in edge (5), has the same curvature as the curves following on from it, the circular arc (7) and the spherical indentation (3) respectively.

3. A socket insert according to claim 1 or 2, **characterised in that** the shape of the curve (14) is sinusoidal or has a polynomial form.

4. A socket insert according to one of claims 1 to 3, **characterised in that** the formula of the curve (14) at its end point (A), the start (12) of the circular arc (7), reads f "_{A} (x, y) - 1/R_{E} and at its end point (B), the run-in edge (5) and the start of the circular arc of the spherical indentation (3), reads f"_{B}(x,y) = 1/R_{K}.

## Revendications

1. Insert cotyloïdien d'une prothèse de hanche dans laquelle la tête sphérique de l'os fémoral est placée dans le creux en forme de calotte de l'insert cotyloïdien, et où cette calotte présente une zone d'entrée qui se termine d'un côté par un arc de cercle de façon tangentielle dans la face frontale de l'insert cotyloïdien et s'étend de l'autre côté jusqu'au bord d'entrée, **caractérisé en ce que**, entre le début (12) de l'arc de cercle (7), avec le rayon (11) de longueur (R_{E}), et le bord d'entrée (5) de la calotte, avec le rayon (13) de longueur (R_{K}), la zone d'entrée (6) forme une courbe (14) dont la fonction est deux fois différentiable en chaque point.

2. Insert cotyloïdien selon la revendication 1, **caractérisé en ce que** la courbe (14), à ses points d'extrémité respectifs (A), à savoir le point d'extrémité (12) de l'arc de cercle (7), et (B), à savoir le bord d'entrée (5), présente la même courbure que les courbes qui s'y raccordent, à savoir l'arc de cercle (7), respectivement la calotte (3).

3. Insert cotyloïdien selon la revendication 1 ou 2, **caractérisé en ce que** le tracé de la courbe (14) est sinusoïdal ou présente une forme de polynôme.

4. Insert cotyloïdien selon l'une quelconque des revendications 1 à 3, **caractérisé en ce que** la formule de la courbe (14), à son point d'extrémité (A), à savoir le début (12) de l'arc de cercle (7), est f'_{A}(x,y) = 1/R_{E}, et à son point d'extrémité (B), à savoir le bord d'entrée (5) et le début de l'arc de cercle de la calotte (3), est f''_{B}(x,y) = 1/R_{K}.
